(19) **Europäisches Patentamt / European Patent Office / Office européen des brevets**

(11) **EP 2 606 916 A1**

(12) **EUROPEAN PATENT APPLICATION**
published in accordance with Art. 153(4) EPC

(43) Date of publication:
**26.06.2013 Bulletin 2013/26**

(21) Application number: **11818152.8**

(22) Date of filing: **12.08.2011**

(51) Int Cl.:
***A61L 27/00*** *(2006.01)*

(86) International application number:
**PCT/JP2011/068431**

(87) International publication number:
**WO 2012/023510 (23.02.2012 Gazette 2012/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.08.2010 JP 2010184230**

(71) Applicants:
• **Saga University**
  **Saga-shi, Saga 840-8502 (JP)**
• **Kyocera Medical Corporation**
  **Osaka-shi, Osaka 532-0003 (JP)**

(72) Inventors:
• **MAWATARI, Masaaki**
  **Saga-Shi**
  **Saga 840-8502 (JP)**
• **NODA, Iwao**
  **Osaka-shi**
  **Osaka 532-0003 (JP)**

(74) Representative: **HOFFMANN EITLE**
  **Patent- und Rechtsanwälte**
  **Arabellastrasse 4**
  **81925 München (DE)**

(54) **BIOLOGICAL IMPLANT**

(57)     Provided is a bioimplant superior in antimicrobial action and safety in the body. The bioimplant according to the present invention comprises a base material of metal, ceramic, or plastic and a thermal spraying film of a calcium phosphate-based material formed at least partially thereon and the silver concentration in the thermal spraying film is 0.02 wt % to 3.00 wt %.

Fig. 1

EP 2 606 916 A1

**Description**

The present invention relates to an antimicrobial bioimplant.

BACKGROUND OF THE INVENTION

Use of a bioimplant for treatment of bone injuries/diseases is steadily increasing along with expansion of active and elderly populations. For use as a bone substitute for broken or removed bones or for use as a support to assist a weakened bone, the synthetic bone substitute should form a strong joint or bone together with natural bones and assure the structural integrity thereof. A bone can grow into a neighboring tissue, especially when it is a porous tissue similar to the bone. However, in addition to the growth into porous tissue, the natural bone thus grown into the porous tissue should bind to the bioimplant, forming strong adhesion between them.

For solid fixation of a bioimplant in a bone, it is important that the bone grows on the implant surface and/or into the implant. Various studies showed that bioimplants of cobalt-chromium (Co-Cr) alloys and titanium (Ti) alloys carrying a coating of calcium phosphate, such as biological apatite, accelerates bone deposition far more effectively than those carrying no coating. The biological apatite $Ca_{10}(PO_4)_6(OH)_2$ is one of the main compounds constituting human bones and teeth. These synthetic hydroxyapatites (HAs) are very similar to natural apatites and there are studies aimed at using HAs in bioimplants for dental and orthopedic treatments. It is possible to produce a bioimplant that can be integrated easily with neighboring bones and tissues after implantation, by coating with HA or any other crystalline calcium phosphate.

However, although use of the synthetic joints in orthopedics for treatment of degenerative joint diseases is a therapeutic treatment effective for reconstruction of joint function, microbes may proliferate on the surface of the synthetic joints, causing post-operative infection. It is because microbes can adhere to the surface of the synthetic joint and the adhered microbes can form a habitat called biofilm. In such a case, antimicrobial agents (antibiotics) are not effective any more, making it difficult to treat the infection. Moreover if myelitis occurs, it is necessary to remove the synthetic joint and repeat the surgery and there may be possibly a case where the infected limb should be ablated.

Accordingly, proposed are a method of forming a HA layer higher in crystallinity and larger in specific surface area, which is suited for impregnation for example of antibiotics, on the surface of a bioimplant by depositing HA thereon and drying the resulting layer formed and a therapeutic agent-containing bioimplant produced by impregnating an antibiotic into the coating layer (Patent Document 1: JP-A No. 2005-506879). However, although such a bioimplant prepared by the method is suited for impregnation of antibiotics, because the film has a uniform void diameter and a uniform void rate, it is difficult to deliver the drug at a desired speed in the controlled manner, causing a problem that the drug is facilely released rapidly at a constant speed.

Alternatively, the applicant proposed a method of controlling the rate of releasing an antibacterial or antimicrobial agent by adjusting the elution speed of HA by means of regulating the crystallinity of the coating layer of calcium phosphate-based material (Patent Document 2: JP-A No. 2008-73098).

SUMMARY OF THE INVENTION

Antibacterial agents are antimicrobial and at the same time have a problem that they show toxicity to enzymes and membranes in cells by acting them concentration-dependently. For that reason, there exists a need for a bioimplant that is not only superior in antimicrobial action, but also less toxic to tissues and organs in the body, thus higher in safety in the body.

Thus, an object of the present invention is to provide a bioimplant superior in antimicrobial action and also higher in safety in the body.

The bioimplant according to the present invention, which was made to overcome the problems above, is characterized in that it comprises a base material of metal, ceramic or plastic and a thermal spraying film made of a calcium phosphate-based material formed at least partially thereon, wherein the silver concentration in the thermal spraying film is 0.02 wt % to 3.00 wt %.

In the present invention, the calcium phosphate-based material is preferably a compound or a mixture of two or more compounds selected from the group consisting of hydroxyapatite, $\alpha$-tricalcium phosphate, $\beta$-tricalcium phosphate, and tetracalcium phosphate.

In addition, the thickness of the thermal spraying film is preferably 5 to 100 $\mu$m.

When used, the bioimplant according to the present invention is effective in accelerating treatment of infections by the sterilization action due to its high antimicrobial activity. Because it is highly safe in the body, it is possible to use it safely even in less resistant patients, such as compromised hosts (more susceptible hosts), who are growing in number.

BRIEF DESCRIPTION OF THE DRAWINGS

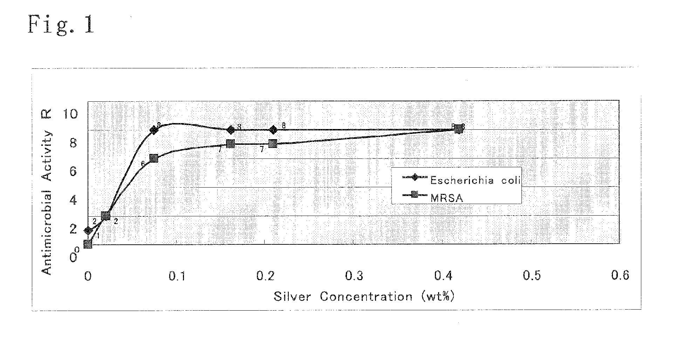

Fig. 1 is a graph showing the relationship between the antimicrobial activity R and the silver concentration in the thermal spraying film of Example 1 of the present invention.

DETAILED DESCRIPTION OF THE PREFERRED EMBODIMENTS

Hereinafter, favorable embodiments of the present invention will be described in detail.
The bioimplant according to the present invention is a bioimplant, comprising a base material of metal, ceramic, or plastic and a thermal spraying film made of a calcium phosphate-based material formed at least partially thereon, wherein the silver concentration in the thermal spraying film is 0.02 wt % to 3.00 wt %.

The bioimplants according to the present invention include metal, ceramic, and plastic implants, such as synthetic bones and fixation devices used for treatment of diseases and injuries, synthetic joints used for reconstruction of lost joint function, and synthetic tooth roots used for reconstruction of teeth.

A metal, ceramic, or plastic material may be used as the base material of the bioimplant. A stainless steel alloy, a cobalt-chromium alloy, titanium, a titanium alloy, alumina, zirconia or the like may be used as the metal, but titanium and titanium alloys are preferable. The titanium alloys for use include alloys of titanium with at least one metal selected from aluminum, tin, zirconium, molybdenum, nickel, palladium, tantalum, niobium, vanadium, platinum and the like. Preferably, it is Ti-6Al-4V alloy. Alternatively, the ceramics for use include, for example, alumina, zirconia, composite alumina-zirconia ceramics and the like. Yet alternatively, the plastics for use include, for example, polyethylenes, fluorine resins, epoxy resins, PEEK resins, Bakelites and the like.

The calcium phosphate-based material for use may be a compound or a mixture of two or more compounds selected from the group consisting of hydroxyapatite, $\alpha$-tricalcium phosphate, $\beta$-tricalcium phosphate, and tetracalcium phosphate. It is preferably hydroxyapatite.

(Production Method)

The thermal-spraying methods used for forming a thermal spraying film of a calcium phosphate-based material include flame-spraying method, high-speed flame-spraying method, plasma-spraying method, and cold spraying method. For example in the case of the flame-spraying method, a film is formed on the surface of a base material by melting a thermal-spraying material or bringing it close to the melting state by placing it in a gas flame generated with oxygen and a flammable gas and spraying the resulting thermal-spraying material, on the base material. In the case of the normal flame-spraying method, the thermal-spraying temperature is about 2700°C and the thermal-spraying speed is Mach 0.6. Under normal thermal-spraying condition, for example, a thermal-spraying powder can be fed with 100 psi dry air into a gas frame torch generated with 50 psi oxygen gas and 43 psi acetylene gas and the resulting powder be thermally sprayed at a thermal-spraying distance of 60 to 100 mm.

The thickness of the thermal spraying film is 5 to 100 $\mu$m, preferably 20 to 40 $\mu$m. It is because it is not possible to cover the thermal-spraying area entirely when the thickness is less than 5 $\mu$m and the adhesion strength of the film declines because of the residual stress during thermal spraying when it is more than 100 $\mu$m.

It is possible to control the silver concentration in the thermal spraying film by adjusting the amount of the raw silver material blended to the thermal-spraying material, i.e., the calcium phosphate-based material. The silver concentration in the thermal spraying film is 0.02 wt % to 3.00 wt %, preferably 0.02 wt % to 2.50 wt %, more preferably 0.02 wt % to 2.00 wt %, and more preferably 0.02 wt % to 1.11 wt %. It is because the antimicrobial action is not sufficient when the silver concentration is less than 0.02 wt %. Alternatively when it is more than 3.00 wt %, the implant may become toxic to tissues and organs in the body. According to literature, use of a great amount of silver leads to Argylia disease (disease leading to graying in color of the entire skin), decrease of leucocytes, and damage to liver and kidney. Our studies also showed that there are deformation of cells and inhibition of neonatal bone formation when the silver concentration is more than 3.00 wt %.

An example of the bioimplant according to the present invention is a synthetic joint consisting of a stem and a neck unit formed on the top end of the stem for fixation of bone head ball, wherein at least part of the surface of the neck unit is covered with a thermal spraying film of a calcium phosphate-based material and the silver concentration in the thermal spraying film is 0.02 wt % to 3.00 wt %. The synthetic joint is preferably made of titanium or a titanium alloy.

[Examples]

Example 1

(Sample Preparation)

Hydroxyapatite containing a particular amount of silver oxide was sprayed onto one side of a pure titanium plate with a size of 50 mm × 50 mm × 2 mm by flame-spraying method, to form a thermal spraying film having a thickness of about 40 μm. Samples having silver concentrations in the thermal spraying film of 0.02, 0.07, 0.16, 0.21, and 0.42 wt % were prepared by varying the amount of the silver oxide added.

The flame spraying was carried out by introducing, with 100 psi dry air, the thermal-spraying powder into a gas frame torch generated with 50 psi oxygen gas and 43 psi acetylene gas and spraying the fused powder at a thermal-spraying distance of 60 to 100 mm.

(Measurement of Silver Concentration)

After sufficient drying at 100°C, each sample was weighed and then dissolved in a nitric acid solution (5 mL of nitric acid and 50 mL of purified water) under heat. The silver concentration in the film was determined by measuring the silver concentration in the solution quantitatively by ICP emission spectrophotometric analysis. Then, the sample after removal of the film by solubilization was dried sufficiently and weighed again, and the film weight was calculated from the difference in weight from the sample before solubilization. The silver concentration in film (wt %) was calculated by dividing the amount of silver in film by the weight of the film.

(Test for Antimicrobial Activity)

The antimicrobial activity was determined by evaluating the antimicrobial activities to Escherichia coli and Methicillin-resistant Staphylococcus aureus (MRSA) in accordance with JIS Z 2801 "Antibacterial products - Test for antibacterial activity and efficacy." However, as the use of the present antimicrobial product in the body is taken into consideration, fetal bovine serum was used as the medium, replacing 1/500 normal bouillon medium, to mimic the environment in the body. The culture temperature was also changed from 35°C to 37°C. The culture was performed in a dark place for 24 hours.

The antimicrobial activity (R), which is a value indicating the difference between the logarithmic values of the viable cell counts on an antimicrobially-processed product and on an unprocessed product after inoculation and incubation of the microbe and is defined by the following Formula:

$$\text{Antimicrobial activity} = \log\left[\frac{\text{average of the viable cell count on unprocessed sample after 24 hours}}{\text{average of the viable cell count on antimicrobially processed sample after 24 hours}}\right]$$

For example, an antimicrobial activity R of 7 indicates that the viable cell count after test is $1/10^7$ of that before test. According to JIS Standard, the antimicrobial activity is considered satisfactory when the value is 2 or more.

Fig. 1 is a graph showing the relationship between the antimicrobial activity R and the silver concentration in a thermal spraying film (wt %). When the silver concentration is 0.02 wt % or more, the antimicrobial activity R is not smaller than 2, indicating that such samples show high antimicrobial activity both to Escherichia coli and MRSA.

Example 2

(Sample Preparation)

Samples having silver concentrations in the thermal spraying film of 0.21, 1.11, 3.48, and 13.03 wt % were prepared similarly to Example 1 by varying the amount of the silver oxide added. Separately, a sample carrying silver oxide-free hydroxyapatite thermally sprayed thereon was prepared as control. The size of the sample was 14 mm in diameter and 1 mm in thickness.

(Cell Adhesion Test)

The cell adhesion test was performed in the following manner: A mouse-derived osteoblast precursor cell line MC3T3-E1 was pre-cultured and inoculated on the sample immersed in $\alpha$-MEM + 10% FBS. After culture under 5% $CO_2$ at 37°C for 2 hours, the cytoskeleton and the nucleus of the cells on the sample were stained by fluorescent staining and the number of the cells thereon were determined and the morphology thereof observed.

There was no significant difference in the number of the adhered cells among the 4 kinds of samples analyzed. However, the morphological observation of the cells showed deformation of the cells on the samples respectively having silver concentrations of 3.48 and 13.03 wt %, which are outside the scope of the present invention. Table 1 shows the cell diameter ratios (%) after test on the samples at respective silver concentrations. The cell diameter ratio after test is the relative ratio to the cell diameter on the sample at a silver concentration of zero. It is the average of the diameters of multiple cells observed in the photograph obtained. The cell diameter declined to 81% when the silver concentration was 3.48 wt % and to 74% when it was 13.03 wt %, indicating that silver shows toxicity to the cells.

[Table 1]

|  | Silver concentration (wt %) | | | | |
| --- | --- | --- | --- | --- | --- |
|  | 0 | 0.21 | 1.11 | 3.48 | 13.0 3 |
| Cell diameter ratio after test (%) | 100 | 100 | 100 | 81 | 74 |

Example 3

(Animal Test 1: Test on Microbial Infection)

A microbial infection test was performed using sample having a silver concentration of 0.21 wt %, which was prepared in a manner similar to Example 1. Separately, a sample carrying silver oxide-free hydroxyapatite thermally sprayed thereon was prepared as control. The size of the sample was 8 mm in diameter and 1 mm in thickness. The microbial infection test was performed in the following manner: The sample described above was embedded under the skin on the back of a male SD-line rat (body weight: 300-350g) under abdominal anesthesia with Nembutal and $1.2 \times 10^6$ CFU of Methicillin-resistant Staphylococcus aureus (MRSA) capable of forming a biofilm, which was isolated from a clinical material, was inoculated on the area. After growth of the rat with normal feed for 72 hours, the sample was separated therefrom and cleaned by ultrasonication (5 minutes) and the cell count in the washing water was determined by plate culture method.

The average cell counts of the MRSA cells (CFU) adhered to the sample were $1.5 \times 10^5$ in the control group and $1.1 \times 10^4$ (P<0.001) in the 0.21 wt % test group. There was observed decrease in cell count on the sample of the present Example, indicating that the sample of this Example shows antimicrobial action effectively even in the body.

Example 4

(Animal Test 2: Endosteal Implant Test)

An endosteal implant test was performed using samples having silver concentrations of 0.21 and 13.03 wt %, which were prepared in a manner similar to Example 1. Separately, a sample carrying silver oxide-free hydroxyapatite thermally sprayed thereon was prepared as control. The size of the sample was 1 mm in diameter and 20 mm in length.

The endosteal implant test was performed in the following manner: A hole was formed on the tibial tuberosity of a male SD-line rat (body weight: 300 to 350 g) under abdominal anesthesia with Nembutal by drilling with a No. 18G needle and the sample described above was placed therein. The rat was killed one month after the surgery and the tibia was collected with the sample, to give its morphological sample. After staining with toluidine blue, the osteogenetic rate was determined by observation under optical microscope. The osteogenetic rate is defined by the following Formula:

$$\text{Osteogenetic rate (\%)} = (\text{Sum of the lengths of osteogenetic region/peripheral length of embedded material} \times 100)$$

The osteogenetic rates were similar between the control group and 0.21 wt % test group, respectively at 73.5% and 74.8%, but the osteogenetic rate of the 13.03 wt % test group showed a low value of 31.7%. It would probably be due to inhibition of neonatal bone formation that is caused by the toxicity of the higher concentration of silver to bone cells.

Example 5

(Cell Toxicity Test)

A cell toxicity test was performed in accordance with ISO10993-5, by using the sample having silver concentration of 0.21 wt % that was prepared in Example 2. Specifically, colony forming tests by extraction method and by direct method were carried out. The extraction method is a method of examining the toxicity of the extract (eluate) from a sample, while the direct method is a test method of directly evaluating the toxicity of the sample surface. A sample carrying silver oxide-free hydroxyapatite thermally sprayed thereon was used as control. The test procedure is as follows:

(Extraction Method)

M05 medium was added in an amount of 1 mL to 6 cm$^2$ of the sample surface area and extraction was performed at 37°C for 24 hours. V79 cells were inoculated on a dish and the extraction solution was added in dilution series. After culture at 37°C for 6 days, the dish was fixed and stained with Giemsa and the colony count was determined, to give the colony-forming rate and IC$_{50}$ (50% lethal dose).

(Direct Method)

A sample was brought into tight contact with the bottom of a dish and V79 cells were inoculated. After culture in MEM10 medium at 37°C for 6 days, the dish was fixed and stained with Giemsa. The colony count was determined, to give the colony-forming rate, which was compared with negative and positive controls.
There was observed no toxicity by the material in the test by extraction method, as the IC$_{50}$ values of the control group and the 0.21 wt % test group were both 100% or more. On the other hand, there was no significant difference in colony-forming rate, as they were respectively 72.4% in the control group and 71.4% in the 0.21 wt % test group by the direct method, and thus there was observed no toxicity presumably caused by silver.

**Claims**

1. A bioimplant comprising a base material of metal, ceramic, or plastic and a thermal spraying film made of a calcium phosphate-based material formed at least partially thereon, the silver concentration in the thermal spraying film being 0.02 wt % to 3.00 wt %.

2. The bioimplant according to Claim 1, wherein said calcium phosphate-based material is a compound or a mixture of two or more compounds selected from the group consisting of hydroxyapatite, α-tricalcium phosphate, β-tricalcium phosphate, and tetracalcium phosphate.

3. The bioimplant according to Claim 1 or 2, wherein the thickness of said thermal spraying film is 5 to 100 μm.

Fig. 1

**INTERNATIONAL SEARCH REPORT**

| International application No. |
|---|
| PCT/JP2011/068431 |

A. CLASSIFICATION OF SUBJECT MATTER
*A61L27/00*(2006.01)i

According to International Patent Classification (IPC) or to both national classification and IPC

B. FIELDS SEARCHED

Minimum documentation searched (classification system followed by classification symbols)
A61L27/00

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2011 |
| Kokai Jitsuyo Shinan Koho | 1971–2011 | Toroku Jitsuyo Shinan Koho | 1994–2011 |

Electronic data base consulted during the international search (name of data base and, where practicable, search terms used)
JSTPlus/JMEDPlus/JST7580(JDreamII), MEDLINE(STN)

C. DOCUMENTS CONSIDERED TO BE RELEVANT

| Category* | Citation of document, with indication, where appropriate, of the relevant passages | Relevant to claim No. |
|---|---|---|
| X | SHIMAZAKI et al., In Vivo Antibacterial and Silver-Releasing Properties of NovelThermal Sprayed Silver-Containing Hydroxyapatite Coating, J Biomed Mater Res Part B: Appl Biomater, 2009, 92B, 386-389 | 1-3 |
| X | NODA et al., Development of Novel Thermal Sprayed Antibacterial Coatingand Evaluation of Release Properties of Silver Ions, J Biomed Mater Res Part B: Appl Biomater, 2008, 89B, 456-465 | 1-3 |
| X | ANDO et al., "Development of antibacterial biomaterials", Orthopaedic Surgery and Traumatology, 30 April 2010 (30.04.2010), vol. 53, no.5, 467-475 | 1-3 |

☒ Further documents are listed in the continuation of Box C.  ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 31 August, 2011 (31.08.11) | 13 September, 2011 (13.09.11) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2011/068431

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2010-65304 A  (Saga University),<br>25 March 2010 (25.03.2010),<br>claims<br>(Family: none) | 1-3 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005506879 A

- JP 2008073098 A